# FASCICULE DE BREVET EUROPEEN

(11) **EP 0 795 107 B1**
(45) Date de publication et mention de la délivrance du brevet: **23.06.1999**
(21) Numéro de dépôt: 95941132.3
(22) Date de dépôt: 23.11.1995
(51) Int. Cl.: F22B 3/04, A61H 33/06

(54) **APPAREIL DE PRODUCTION DE VAPEUR D'EAU**
DAMPFERZEUGER
STEAM GENERATING APPARATUS

(30) Priorité: 28.11.1994 FR 9414561
(43) Date de publication de la demande: 17.09.1997
(73) Titulaire: Luc, Jean-Paul, 13510 Eguilles (FR)
(72) Inventeur: Luc, Jean-Paul, 13510 Eguilles (FR)
(74) Mandataire: Boutin, Antoine
(86) Numéro de dépôt international: FR9501546
(87) Numéro de publication internationale: WO9617208

(56) Documents cités:
- US-A- 4 616 122
- US-A- 5 215 043

## Description

La présente invention concerne un dispositif pour produire de la vapeur d'eau particulièrement adapté pour satisfaire aux besoins des soins corporels par le bain de vapeur.

Le dispositif selon l'invention est particulièrement destiné aux salles d'eau, bains, douches et aux appareils d'hydrothérapie, de soins corporels et de remise en forme.

La production de vapeur est traditionnellement effectuée à l'aide d'un générateur de vapeur (bouilleur) fonctionnant grâce à l'énergie électrique alimentant par exemple une résistance de chauffage, tel que par exemple décrit dans le brevet US4616122 (BURIAN et al).

L'utilisation de l'énergie électrique dans le cadre d'une cabine de douche placée dans une salle de bains pose des problèmes pour garantir l'utilisateur contre tout risque d'électrocution ou choc électrique, et par ailleurs engendre des coûts importants du fait de la complexité d'un tel générateur.

Le dispositif selon l'invention permet de remédier à cette inconvénient en utilisant directement et uniquement l'eau chaude sous pression produite par exemple par un chauffe eau tel que ceux installés dans les habitations.

Le brevet US5215043 (TSUTSUMI) décrit un générateur de vapeur pour bains de vapeur, qui produit de la vapeur d'eau à partir d'eau chaude.

Ce générateur comprend une enveloppe ou partie principale fermée, qui est munie d'un orifice d'alimentation en eau chaude, d'un orifice d'entrée d'air, d'un orifice d'évacuation d'eau chaude, et d'un orifice de sortie d'un mélange d'air et de vapeur d'eau.

La production de vapeur d'eau est obtenue par mise en contact de l'air avec l'eau chaude, à l'intérieur de l'enveloppe.

Ce document décrit plusieurs dispositifs permettant de mettre en contact l'air et l'eau chaude, à savoir : des plaques obliques superposées, des tubes ailetés, ou une structure tridimensionnelle, sur lesquels l'eau chaude ruisselle ; deux autres modes de réalisation décrits dans ce document utilisent une buse d'aspersion disposée respectivement en partie inférieure et en partie supérieure de l'enveloppe.

L'objet de l'invention est de procurer un générateur de vapeur d'eau amélioré.

En effet, le générateur, décrit dans le document TSUTSUMI, présente plusieurs inconvénients : tout d'abord, il semble qu'un ventilateur (qui, s'il doit être raccordé à une source d'énergie électrique, a le même inconvénient que celui sus-cité) soit nécessaire pour accélérer la circulation de l'air ; un autre inconvénient très important est l'encombrement élevé des appareils décrits ; un troisème inconvénient consiste en ce que le mélange d'air et de vapeur d'eau produite, est délivré à la sortie de l'appareil à une température très inférieure à celle de l'eau chaude alimentant l'appareil ; un autre inconvénient découlant de celui-ci est que cet appareil doit être alimenté en eau chaude à une température élevée, voisine de 70 à 80° centigrades.

La solution au problème posé consiste à procurer un appareil produisant un mélange d'air et de vapeur d'eau à partir d'une arrivée d'eau chaude sous pression constitué par un boîtier équipé d'une buse de pulvérisation reliée par un conduit à une arrivée d'eau chaude, un orifice d'évacuation d'eau, un orifice d'entrée d'air, disposé au niveau de ladite buse, et un orifice de sortie de vapeur et dans lequel l'agencement des parois du boîtier définit une chambre de pulvérisation dont une partie importante est occupée par le jet d'eau chaude pulvérisée par la buse, au moins une paroi, interne au boîtier et disposée à proximité de ladite buse, interceptant une partie substantielle dudit jet pour favoriser la création d'un brouillard d'eau chaude.

De préférence cet appareil générateur de vapeur d'eau comporte, à l'intérieur d'un boîtier :
- une chambre ou cavité de pulvérisation et de mélange, au travers duquel circule l'air,
- une ou plusieurs buses ou gicleurs, comportant un ou plusieurs orifices de pulvérisation, alimentées en eau chaude, qui produit (produisent) un (ou plusieurs) jet(s) qui est (sont) de préférence volumiques ou tridimensionnel(s), par opposition notamment aux jets plats ou aux jets dit "solides" ou pleins ou rectilignes, de petites gouttelettes dont la distribution est de préférence sensiblement uniforme à l'intérieur du jet, laquelle buse délivre de préférence un jet dit à cône plein,
- un ou plusieurs déflecteurs ou parois ou écrans, de préférence sensiblement plans, disposés à proximité (à faible distance) de la buse, pour intercepter une partie substantielle du jet.

Le volume (par exemple le cône) occupé par le jet représente (occupe) une partie (portion) importante du volume de la cavité de pulvérisation et de mélange, et les orifices d'entrée et de sortie d'air et de vapeur d'eau de la cavité sont grands, c'est-à-dire, ont une section du même ordre de grandeur que la section de passage ou section transversale de la cavité.

Selon un mode préféré de réalisation de l'invention, l'appareil comporte :
- une (première) chambre d'admission d'air, sensiblement de révolution, d'axe vertical, de forme générale cylindrique ou tronconique,
- une deuxième chambre de pulvérisation d'eau, et de mélange et de contact entre l'eau et l'air, sensiblement de révolution, d'axe vertical, de forme générale cylindrique ou tronconique,
- lequelles première et deuxième chambres communiquent par un premier passage ou restriction au travers duquel peut circuler l'air,
- une (ou plusieurs) paroi pourvue d'une face dite "active" -c'est à dire dirigée vers le jet pulvérisé - formant écran ou cible, c'est à dire interceptant une partie substantielle (par exemple la totalité) du jet, sensiblement plane, laquelle paroi est en forme de disque, laquelle paroi est sensiblement perpendiculaire à l'axe longitudinal (de révolution et/ou de symétrie) de la deuxième cavité ou chambre (de pulvérisation), et est située à l'intérieur de celle-ci,
- une ou plusieurs buses raccordées à un orifice d'entrée d'eau chaude délivrant un jet dit conique, c'est à dire contenu dans une enveloppe de forme conique, laquelle buse est un pulvérisateur (axial ou tangentiel) à cône plein, c'est à dire assurant une distribution/répartition de l'eau pulvérisée sensiblement uniforme dans une section du jet perpendiculaire à l'axe de révolution de la forme (l'enveloppe) conique du jet, lequel jet est sensiblement vertical, ascendant (dirigé du bas vers le haut de la chambre) et assure une dispersion des gouttelettes selon un angle au moins égal à 45° et au plus égal à 120° (par exemple égal à 60° ou 90°),
- l'orifice de sortie de ladite buse est situé dans ladite chambre d'admission d'air,
- laquelle paroi , en forme générale de disque formant écran, est sensiblement horizontale et est disposée en regard de l'orifice de sortie d'eau de la buse et à une distance de celui-ci qui est telle que le rapport du rayon du disque à ladite distance est compris entre 0.3 et 3 de préférence voisin de 0.5 à 1.8,
- lequel premier passage ou restriction est délimité par une paroi (de préférence de révolution) ou lèvre de guidage disposée entre la buse et la paroi déflectrice, et séparant lesdites première et deuxième chambres,
- lequel appareil est doté d'un (ou plusieurs, de préférence régulièrement répartis) orifice d'entrée d'air dans ladite chambre d'admission qui est (sont) de grande section, qui s'étend(ent) de préférence sur une partie substantielle du pourtour du boîtier de l'appareil (sur une partie substantielle de sa circonférence dans le cas d'un boîtier cylindrique), par exemple sur une portion d'au moins un quart du pourtour.

La section (aire) de l'orifice d'entrée d'air est de préférence nettement supérieure à la section de l'orifice de sortie du mélange air - vapeur, par exemple voisine du double ou du triple de la section de l'orifice de sortie.

L'invention permet d'obtenir des appareils performants, très compacts (dont l'encombrement hors tout sera par exemple voisin de 2 à 3 dm³), très simples et peu coûteux à fabriquer, qui ne nécessitent pas d'alimentation électrique.

La configuration particulière du jet de gouttelettes, de l'écran, de la chambre de pulvérisation et des orifices d'entrée et de sortie d'air, favorise la circulation "naturelle" de l'air, et favorise la production d'un brouillard (ou vapeur) d'eau et son mélange avec l'air pénétrant par l'orifice d'entrée.

L'invention permet d'obtenir en sortie de l'appareil un mélange d'air et de vapeur d'eau dont la température est très voisine de la température de l'eau chaude alimentant l'appareil (en général inférieure de 1 à 5°C seulement par rapport à celle ci), on peut donc alimenter l'appareil avec de l'eau dont la température est inférieure à 60°C, par exemple de 45 à 50°C (et bien sur au delà).

Grâce au très faible volume de l'appareil, celui ci a une inertie (ou masse) thermique faible ce qui permet d'obtenir de la vapeur quelques secondes seulement après le début de l'alimentation en eau chaude.

Dans le cas où l'eau chaude délivrée à l'appareil est distribuée par l'intermédiaire d'un robinet (mélangeur) thermostatique, l'utilisateur peut simplement et précisément régler la température du bain de vapeur.

Grâce à l'invention on obtient, un procédé de production d'un mélange d'air et de vapeur d'eau dans lequel :
- on alimente, de préférence sous une pression relative supérieure ou égale à 1 bar (10⁵ Pascal) par exemple voisine de 2 à 3 bars, en eau chaude (c'est à dire de l'eau dont la température est au moins égale à 45°C, par exemple comprise entre 50°C et 70°C) une buse de pulvérisation de manière à provoquer (établir) un jet de gouttelettes (micro gouttes) d'eau chaude -dont la dimension moyenne est inférieure à 3 mm, de préférence de l'ordre de 0.1 mm à 2 mm- qui est doté d'une symétrie (de préférence axiale de révolution),
- on dispose une paroi (écran) sensiblement plane, placée sensiblement perpendiculairement à la direction (axe principal de symétrie) du jet, à une distance de l'orifice de sortie de la buse qui est choisie pour qu'une partie substantielle (de préférence sensiblement la totalité) du jet soit intercepté (dévié) par la paroi, favorisant notamment la création et/ou la séparation d'un brouillard d'eau chaude, c'est à dire de gouttelettes ou particules d'eau dont la dimension moyenne est généralement inférieure à (ou de l'ordre de) 0.1 mm,
- on facilite (provoque) une circulation ascendante d'air notamment par des ouvertures de grandes dimensions, et on facilite un contact de l'air avec le jet émis par la buse, et de préférence avec l'écoulement d'eau résultant de l'interception du jet par la paroi.

Dans un appareil selon l'invention, la circulation d'air et le mélange d'air et de vapeur d'eau est facilité par les éléments suivants :
- l'air est réchauffé par l'eau chaude et par conséquent mis en circulation verticale ascendante par un effet de circulation "naturelle" telle que celle observée dans une cheminée (phénomène de "tirage"),
- les orifices d'entrée d'air de grandes dimensions ainsi que les orifices de sortie du mélange d'air et de vapeur d'eau de grandes dimensions, et les dimensions relativement importante de la veine de circulation de l'air et du mélange d'air et de vapeur d'eau,
- le passage de l'air dans une première chambre ou chambre d'admission communiquant avec l'extérieur du boîtier par les orifices d'entrée d'air,
- le passage ultérieur de l'air par un premier passage ou restriction, de section substantielle mais généralement inférieure à la section de passage dans la première chambre, et au travers duquel passe notamment la partie inférieure du jet délivré par la buse,
- le passage, le mélange et la mise en contact de l'air, dans une deuxième chambre ou chambre de pulvérisation, avec les gouttelettes de diverses tailles du jet direct et des particules d'eau de petites dimensions (de brouillard) formée par le jet incident (direct) et les jets secondaires (ou reflechis) issus de la reflexion ou du rebondissement sur la paroi formant écran du jet direct,
- le passage du mélange d'air et de vapeur d'eau dans une restriction prévue de préférence à la périphérie de la paroi formant écran (en forme de disque), jusqu'à une chambre de sortie communiquant avec l'orifice de sortie et d'évacuation du mélange d'air et de vapeur d'eau.

Les nombreux avantages procurés par l'invention seront mieux compris au travers de la description suivante qui se réfère aux dessins annexés, qui illustrent sans aucun caractère limitatif des modes préférentiels de réalisation de l'invention.

La figure 1 illustre en vue en coupe par un plan vertical de symétrie de l'appareil, un premier mode de réalisation d'un générateur de vapeur d'eau selon l'invention.

La figure 2 illustre dans un plan de coupe vertical passant par l'axe 11 général de symétrie ou de révolution d'un appareil, un mode préféré de réalisation de l'appareil selon l'invention.

Les figures 3 et 4 sont des vues en coupe par des plans horizontaux respectivement selon III.III et IV.IV de la figure 2.

La figure 5 est une vue schématique en perspective de l'appareil des figures 2 à 4.

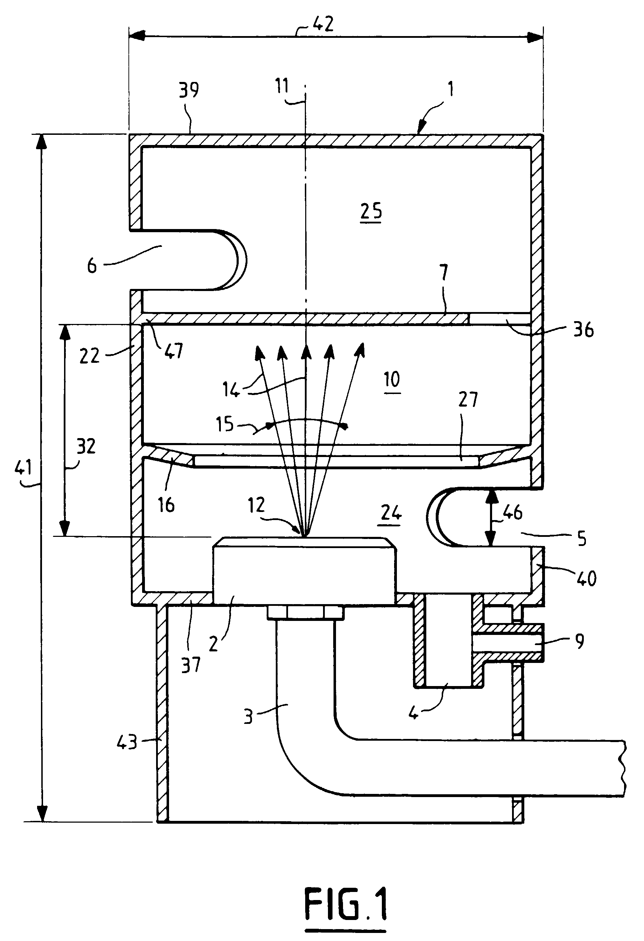

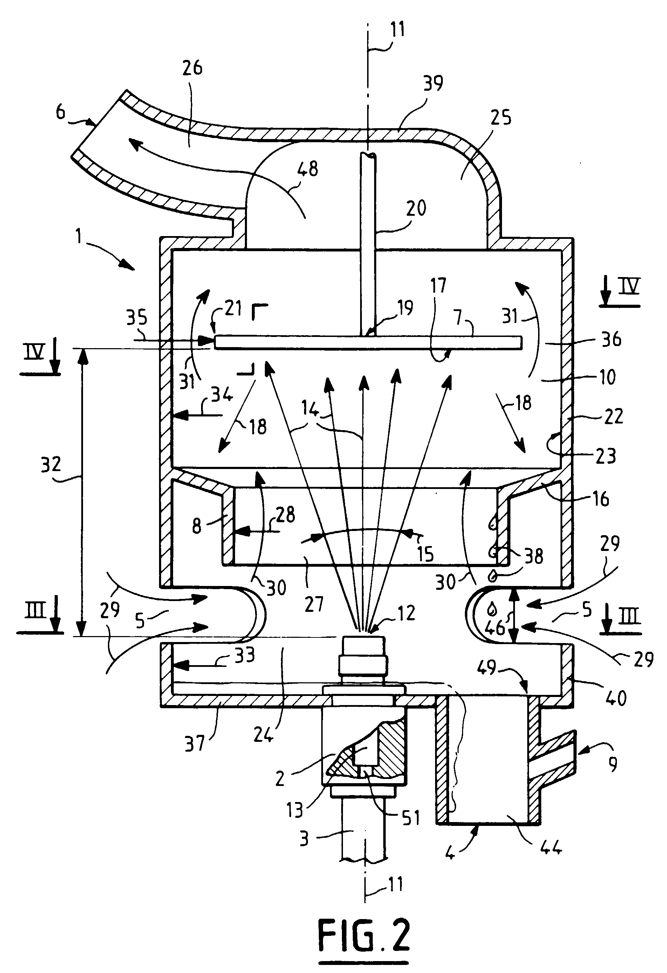

Par référence aux figures 1 et 2, le dispositif comporte un boîtier 1 de forme générale cylindrique, équipé d'une buse de pulvérisation 2, alimentée en eau chaude par un conduit 3, avec un orifice d'évacuation d'eau 4, un orifice d'admission d'air 5, un orifice de sortie de vapeur 6, une paroi intérieure 7, une lèvre intérieure 8, 16 un orifice d'injection d'eau froide 9, une tige de fixation 20.

La pulvérisation d'eau sous forme de microgouttelettes, réalisée par la buse 2, alimentée en eau chaude sous pression par le conduit 3, produit de la vapeur d'eau mélangée aux microgouttelettes. Ce mélange de vapeur d'eau et de microgouttelettes, qui est canalisé dans le boîtier, se sépare lorsque la pulvérisation rencontre la paroi intérieure 7 qui arrête les microgouttelettes en ne laissant s'échapper que la vapeur. Cette vapeur est poussée jusqu'à l'orifice de sortie de vapeur 6 par le flux d'air frais qui pénètre par l'orifice d'admission d'air 5 et s'accélère au passage du retrécissement formé par la lèvre 8, 16 ainsi qu'au passage entre la paroi 7 et le boîtier 1, ce dernier accentuant encore la vitesse d'écoulement du flux. L'eau chaude utilisée s'écoule ensuite le long de la paroi du boîtier 1 jusqu'à ce qu'elle rencontre la lèvre 8, 16 dont les fonctions sont aussi de participer à la production de vapeur en créant un rideau d'eau chaude devant (traversant) le flux d'air frais (entrant dans le boitier) ainsi que d'empêcher cette même eau chaude de sortir par l'orifice d'admission d'air 5, afin de s'évacuer par l'orifice 4.

Une injection d'eau froide par l'orifice 9 peut être réalisée sur l'orifice 4 d'évacuation d'eau chaude afin de la refroidir et d'éviter ainsi tout risque de brûlure dans le cas où celle ci rejetée au contact direct de l'utilisateur.

A titre d'exemple non limitatif, le boîtier de la figure 1 aura des dimensions de l'ordre de 12 cm pour la hauteur et de 10 cm pour son diamètre.

Par référence à la figure 1 particulièrement, l'appareil selon l'invention comporte un boîtier 1 cylindrique s'étendant verticalement parallèlement à l'axe 11 de la buse 2 (et de symétrie du jet 14).

Le boîtier 1 est essentiellement constitué par une paroi supérieure 39, une paroi latérale cylindrique 22, et une paroi inférieure 37.

La paroi cylindrique 22 est percée, dans sa partie inférieure 40, d'ouvertures ou orifices 5 permettant l'entrée de l'air extérieur au boîtier à l'intérieur de celui ci comme représenté par les flèches 29 sur la figure 2.

Dans le mode de réalisation de la figure 1, l'orifice 6 d'évacuation du mélange d'air et de vapeur d'eau est prévu dans la partie supérieure de la paroi cylindrique 22, alors que dans le mode de réalisation représenté figure 2, l'orifice 6 constitue l'extrémité d'un canal ou conduit 26 d'évacuation qui communique avec la partie supérieure de l'enceinte délimitée par le boîtier.

Dans le mode de réalisation représenté figure 2, la buse 2 dont la partie inférieure est raccordée à une canalisation 3 d'arrivée d'eau chaude, s'étend au travers d'un orifice prévu dans le fond 37 du boîtier, en position centrale, l'axe longitudinal 11 de la buse 2 s'étendant selon l'axe longitudinal de la paroi cylindrique 22 du boîtier.

Dans le mode de réalisation représenté figure 1, la buse 2 s'étend au travers d'un orifice prévu également dans le fond 37 du boîtier, qui est décalé par rapport au centre du fond, de sorte que l'axe 11 de la buse est légèrement décalé par rapport à l'axe de symétrie de la paroi cylindrique 22.

Dans les deux modes de réalisation, l'orifice 12 de la buse 2 (situé en partie supérieure de celle ci) prévu à l'intérieur du boîtier 1, délivre un jet schématiquement représenté par des flèches 14 qui est dispersé selon un angle 15 de dispersion de jet.

Le jet direct 14 émis par la buse 2 par son orifice 12, s'étend à l'intérieur d'une enveloppe de forme conique dont le sommet est voisin de l'orifice 12 de la buse et ayant pour axe de révolution ledit axe 11 de la buse.

De préférence, les buses utilisées dans les appareils selon l'invention sont des buses formant des jets à cône plein préférentiellement aux jets de pulvérisation à cône creux.

De telles buses délivrant un jet à cône plein, c'est à dire dans lesquels la distribution de gouttelettes est sensiblement uniforme à l'intérieur du jet, comportent généralement un moyen de mise en rotation de la veine liquide, à l'intérieur de la chambre 13 de la buse ; ceci peut être obtenu, dans le cas de buses axiales, c'est à dire dont l'orifice 51 d'entrée d'eau dans la chambre 13 est disposé sur l'axe longitudinal 11 de la buse 2 (et de la chambre 13) et donc aligné avec l'orifice 12 de sortie, comme c'est le cas sur les figures, par la présence dans la chambre 13 de déflecteurs (non représentés) constituant une grille d'aubes (fixes) parfois appelée hélice ; dans le cas de buses tangentielles, cette rotation du liquide circulant dans la chambre 13 est obtenue par la forme et la disposition du conduit de raccordement entre l'orifice d'entrée 51 (disposé radialement par référence à l'axe 11) et la chambre 13.

Comme schématiquement représenté sur la figure 2, les jets émis par la buse 2 selon les flèches 14 frappent la face inférieure 17 d'une paroi 7 formant un écran ou déflecteur, ce qui provoque le rebondissement d'une partie des gouttelettes (schématiquement représenté par les flèches 18 sur cette figure) qui forment un jet dit réfléchi.

Le même phénomène est également obtenu dans le mode de réalisation la figure 1, et cette interaction du jet et du déflecteur 7 favorise la formation et/ou la séparation de particules d'eau de petites dimensions (constituant un brouillard ou vapeur d'eau) qui se mélangent avec (et sont entrainées par) l'air circulant à travers le boîtier.

Les gouttelettes de grosses dimensions résultant de cette impact du jet 14 sur l'écran 7, sont récupérées ou canalisées par une lèvre 16 de forme sensiblement annulaire en forme de collerette tronconique (dont la face supérieure est incliné vers le centre où l'axe 11 de symétrie), provoquant l'écoulement des gouttes (repérées 38 sur la figure 2) jusqu'à atteindre le bord intérieur de ladite collerette 16 dans le cas de la figure 1, et, dans le cas de la réalisation de la figure 2, jusqu'à atteindre et couler le long de la paroi intérieure d'un tronçon tubulaire 8 cylindrique d'axe 11 délimitant un passage 27 (ou restriction), au travers duquel circule l'air (selon les flèches 30) ainsi que le jet 14 émis par la buse.

Les gouttes 38 d'eau de relativement grandes dimensions ainsi collectées tombent sur le fond 37 du boîtier et sont évacuées par le canal d'évacuation 44, qui comme représenté aux figures 1 à 3, s'étend parallèlement à l'axe 11 entre un orifice 49 prévu dans le fond 37 et un orifice de sortie 4 de l'eau chaude à évacuer.

Par référence à la figure 2 particulièrement, on voie qu'ainsi le boîtier délimite une chambre d'admission 24, à l'intérieur de laquelle l'air pénètre selon les flèches 29 en traversant les orifices d'entrée 5, en partie inférieure et au centre de laquelle est prévu l'orifice de sortie 12 de la buse 2.

L'air ayant pénétré dans ladite chambre 24 prévue en partie inférieure du boîtier 1 s'écoule de manière ascendante jusqu'à la chambre 10 dite de pulvérisation ou de mélange ou de contact entre le jet et l'air, après avoir traversé, comme schématiquement représenté par les flèches 30, une première restriction 27 (ou passage) délimitée par lesdites lèvres 16 dans le cas de la figure 1 ainsi que ledit manchon ou lèvre 8 cylindrique dans le cas de la figure 2.

Le mélange d'air et de vapeur d'eau généré dans la chambre 10 poursuit son écoulement ascendant selon les flèches 31 en passant au travers d'une deuxième restriction (ou passage par exemple annulaire) 36 ménagée entre le bord (ou périphérie) 21 de l'écran 7 et la paroi cylindrique 22, pour rentrer dans une chambre 25 de sortie prévue en partie supérieure du boîtier 1.

Comme représenté schématiquement sur la figure 2, le disque 7 est fixé à la paroi supérieure 39 du boîtier 1 par une tige 20 s'étendant selon l'axe 11 et fixée à son extrémité inférieure à la partie centrale 19 du disque 7.

Dans le mode de réalisation de la figure 1, le déflecteur 7 peut être de forme non cylindrique, et est fixé à la paroi 22 par une partie 47 de sa périphérie ; ce déflecteur délimite avec la paroi cylindrique 22, la deuxième restriction 36 permettant le passage, jusqu'à la chambre 25 de sortie, du mélange d'air et de vapeur d'eau produit dans la chambre 10.

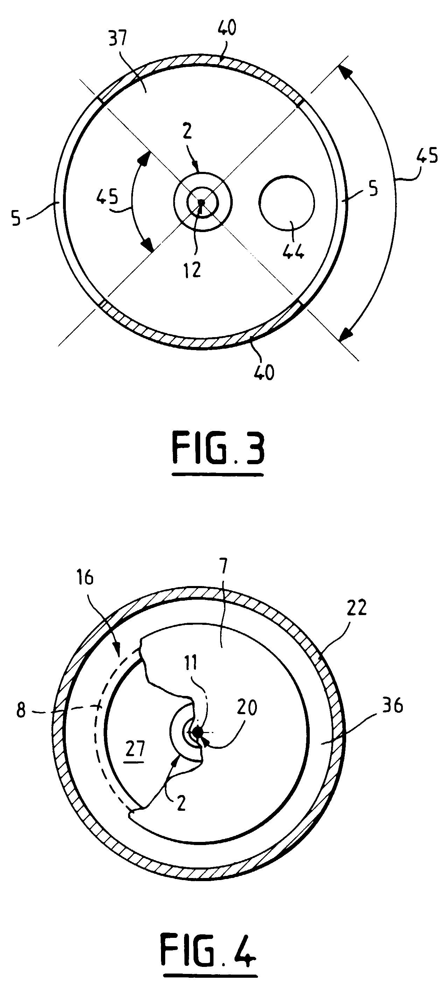

Dans un exemple de réalisation selon le mode préféré de mise en oeuvre de l'invention représenté figure 2 à 4, on a notamment choisi, pour le principaux constituants de l'appareil, les dimensions suivantes : le diamètre intérieur 34 de la paroi cylindrique 22 limitant la chambre 10 (ainsi que le diamètre 33 de la chambre d'admission 24) était de 9,5 cm environ ; le diamètre 35 du disque 7 était de 7,5 cm environ ; la hauteur 46 des orifices 5 d'entrée d'air en forme de lumière s'étendant sur le pourtour de la partie inférieure du boîtier était de 1,5 cm environ ; comme représenté figure 3, l'appareil comportait 2 orifices 5 identiques diamétralement opposés, s'étendant selon un angle au centre 45 voisin de 90° ; ces 2 orifices définissaient une section de passage ou section totale d'entrée d'air de l'ordre de 22 cm².

Le rayon 28 de la face interne de la lèvre 8 définissait une section de passage 27 (perpendiculairement à l'axe 11) de l'ordre de 30 cm².

La section du passage libre 36 était de l'ordre de 40 cm² pour une section de la chambre 10 de mélange de l'ordre de 70 cm² et une section de l'orifice 6 de sortie du mélange d'air et de vapeur d'eau de l'ordre de 7 cm².

La distance 32, mesurée selon l'axe 11, séparant l'orifice 12 de la buse, de la face 17 du disque 7 était de 7,5 cm environ ; le boitier 1 était disposé sur un socle 43 cylindrique similaire à celui de la figure 1 ; l'appareil avait une hauteur totale 41 de l'ordre de 23 cm et un diamètre 43 de l'ordre de 10 cm.

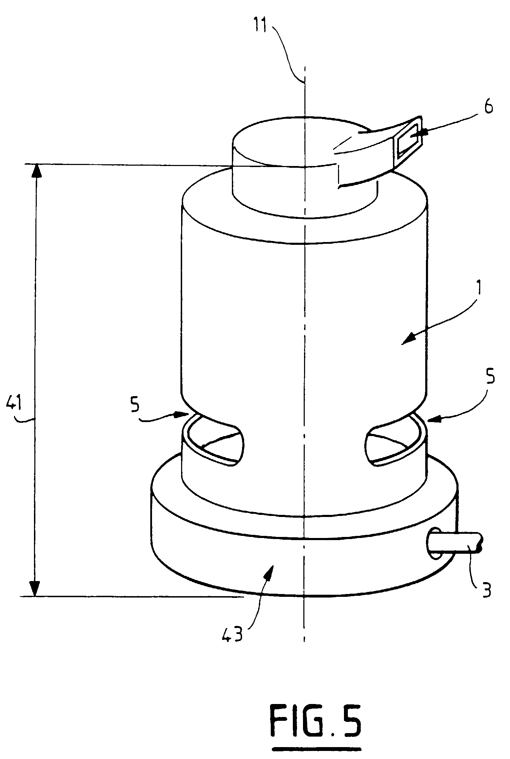

Des essais ont été effectués avec cet appareil équipé de buses distribuée par la société LECHLER, Ulmer strasse 128, D-7430, METZINGEN, ALLEMAGNE, l'appareil défini ci-dessus et correspondant au mode de réalisation des figures 2 à 5 était placé dans une cabine individuelle de douche dont les parois en matière plastique définissaient une enceinte fermée d'un volume voisin d'un mètre cube.

La température extérieure à la cabine était de 20°C.

Dans les essais, on a utilisé une buse (de la société LECHLER sus mentionnée) délivrant un jet à cône plein, proposé par cette société sous la référence série SZ0, et délivrant un jet (direct) 14 dont l'angle d'ouverture (de dispersion) 15 était de 60°.

Pour chacun des essais 1, 2, 3, figurent dans le tableau ci-après les conditions propres à chaque essai ainsi que les températures mesurées à l'intérieur de la cabine de douche dans laquelle était placé le générateur de vapeur précité en fonction du temps écoulé à partir du début de l'alimentation en eau chaude du générateur.

Dans le tableau ci-après, le débit d'eau est exprimé en litre par minute, la pression relative en bar, et les températures en degré Celsius.

| **CONDITIONS DES ESSAIS TEMPERATURE A L'EXTERIEUR DE LA CABINE : 20° C** | | | |
|---|---|---|---|
| N° ESSAI | 1 | 2 | 3 |
| DEBIT D'EAU | 3.1 | 2.3 | 4.1 |
| PRESSION D'EAU | 2 | 3 | 4 |
| Température d'entrée d'eau | 57° | 58° | 59° |
| Température de sortie de vapeur | 56° | 57° | 57° |

| **TEMPS** | **TEMPERATURE INTERIEURE** | | |
|---|---|---|---|
| Après 1 minute | 24° | 22° | 26° |
| Après 2 minutes | 31° | 27° | 35° |
| Après 3 minutes | 36° | 32° | 40° |
| Après 4 minutes | 39° | 36° | 44° |
| Après 5 minutes | 41° | 38° | 45° |
| Après 7 minutes | 41° | 39° | 46° |

## Revendications

1. Appareil pour produire de la vapeur à partir d'une arrivée d'eau chaude sous pression, constitué par un boîtier (1) équipé d'une buse de pulvérisation (2) reliée par un conduit (3) à une arrivée d'eau chaude, un orifice d'évacuation d'eau (4), un orifice d'entrée d'air (5), disposé au niveau de ladite buse, et un orifice de sortie de vapeur (6), les parois du boîtier (1) définissant une chambre de pulvérisation (10) dont une partie importante est occupée par le jet (14) produit par la buse de pulvérisation (2), caractérisé en ce qu'au moins une parois (7), interne au boîtier (1) et disposée à proximité de ladite buse intercepte une partie substantielle dudit jet (14) pour favoriser la création d'un brouillard d'eau chaude.

2. Appareil selon la revendication 1 caractérisé en ce que la paroi (7) interceptant le jet (14) est sensiblement horizontale et disposée en regard de l'orifice de sortie d'eau de ladite buse (2).

3. Appareil selon la revendication 2 caractérisé en ce que la paroi (7) interceptant le jet (14) est en forme de disque et en ce que le rapport de son rayon à la distance la séparant de la buse est compris entre 0,3 et 3 et de préférence voisin de 0,5 à 1,8.

4. Appareil selon les revendications 1 à 3 caractérisé en ce que le boîtier comporte un passage à restriction (27) définissant une chambre d'admission de l'air (24) et la chambre de pulvérisation (10).

5. Appareil selon la revendication 4, caractérisé en ce que le passage à restriction est une lèvre de guidage (8,16) disposée entre la buse de pulvérisation (2) et la paroi déflectrice (7).

6. Appareil selon la revendication 5 dans lequel ladite lèvre (8, 16) est de révolution.

7. Cabine de douche équipée d'un appareil selon l'une quelconque des revendications 1 à 6.

8. Utilisation d'un appareil selon l'une quelconque des revendications 1 à 6 dans laquelle on alimente la buse par de l'eau dont la température est inférieure à 60°.

## Patentansprüche

1. Vorrichtung zum Erzeugen von Dampf, ausgehend von einer Leitung zur Zufuhr von warmem Wasser unter Druck, wobei die Vorrichtung aus einem Gehäuse (1) besteht, das mit einer Zerstäubungsdüse (2), die über einen Kanal (3) mit einer Warmwasserzuführleitung verbunden ist, mit einer Wasserauslaßöffnung (4), einer Lufteinlaßöffnung (5), die auf dem Niveau der Düse angeordnet ist, und mit einer Dampfauslaßöffnung (6) ausgestattet ist, wobei die Wände des Gehäuses (1) eine Zerstäubungskammer (10) festlegen, von der ein großer Teil von dem Strahl (14) eingenommen wird, der von der Zerstäubungsdüse (2) erzeugt wird, **dadurch gekennzeichnet**, daß zumindest eine Wand (7), die im Inneren des Gehäuses (1) und in der Nähe der Düse angeordnet ist, einen beträchtlichen Anteil des Strahls (14) abfängt, um die Erzeugung eines Warmwassernebels zu begünstigen.

2. Vorrichtung nach Anspruch 1, dadurch gekennzeichnet, daß die Wand (7), die den Strahl (14) abfängt, im wesentlichen horizontal verläuft und gegenüber der Wasseraustrittsöffnung der Düse (2) angebracht ist.

3. Vorrichtung nach Anspruch 2, dadurch gekennzeichnet, daß die Wand (7), die den Strahl (14) abfängt, in Form einer Scheibe ausgebildet ist und daß das Verhältnis des Radius derselben zum Abstand, der sie von der Düse trennt, im Bereich von 0,3 bis 3 und vorzugsweise bei etwa 0,5 bis 1,8 liegt.

4. Vorrichtung nach den Ansprüchen 1 bis 3, dadurch gekennzeichnet, daß das Gehäuse einen eingeengten Durchlaß (27) umfaßt, der eine Lufteinlaßkammer (24) und die Zerstäubungskammer (10) festlegt.

5. Vorrichtung nach Anspruch 4, dadurch gekennzeichnet, daß es sich bei dem eingeengten Durchlaß um eine Führungslippe (8, 16) handelt, die zwischen der Zerstäubungsdüse (2) und der Ablenkwand (7) angebracht ist.

6. Vorrichtung nach Anspruch 5, bei der die Lippe (8, 16) eine Drehfläche ist.

7. Duschkabine, die mit einer Vorrichtung nach einem der Ansprüche 1 bis 6 ausgestattet ist.

8. Verwendung einer Vorrichtung nach einem der Ansprüche 1 bis 6, wobei man die Düse mit Wasser speist, dessen Temperatur weniger als 60° beträgt.

## Claims

1. An apparatus for producing steam from an input of hot water under pressure, formed by a casing (1) equipped with a spray nozzle (2) connected by a pipe (3) to a hot-water input, a water discharge orifice (4) and an air inlet orifice (5) arranged at the level of said nozzle, and a steam outlet orifice (6), the walls of the casing (1) defining a spraying chamber (10) a major part of which is occupied by the jet (14) produced by the spray nozzle (2), characterised in that at least one wall (7), internal to the casing (1) and arranged close to said nozzle, intercepts a substantial part of said jet (14) to promote the creation of a mist of hot water.

2. An apparatus according to Claim 1, characterised in that the wall (7) intercepting the jet (14) is substantially horizontal and arranged opposite the water outlet orifice (2) of said nozzle (2).

3. An apparatus according to Claim 2, characterised in that the wall (7) intercepting the jet (14) is disc-shaped and that the ratio of its radius to the distance separating it from the nozzle is between 0.3 and 3, and preferably close to 0.5 to 1.8.

4. An apparatus according to Claims 1 to 3, characterised in that the casing comprises a restricted passage (27) defining an air inlet chamber (24) and the spraying chamber (10).

5. An apparatus according to Claim 4, characterised in that the restricted passage is a guide lip (8, 16) arranged between the spray nozzle (2) and the deflecting wall (7).

6. An apparatus according to Claim 5, wherein said lip (8, 16) is a surface of revolution.

7. A shower cubicle equipped with an apparatus according to any one of Claims 1 to 6.

8. The use of an apparatus according to any one of Claims 1 to 6, in which the nozzle is fed with water, the temperature of which is lower than 60°.
